# EUROPEAN PATENT SPECIFICATION

(11) **EP 3 515 899 B1**
(45) Date of publication and mention of the grant of the patent: **01.09.2021**
(21) Application number: 17777220.9
(22) Date of filing: 21.09.2017
(51) Int. Cl.: C07D 317/22, C10L 1/185, C10L 10/08

(54) **SOLKETAL-ETHERS, PRODUCTION METHOD AND USES THEREOF**
SOLKETAL-ETHERS, HERSTELLUNGSVERFAHREN UND VERWENDUNGEN DAVON
ÉTHERES DU SOLKETAL, LEUR PROCEDE DE PRODUCTION ET LEUR UTILISATIONS

(30) Priority: 21.09.2016 EP 16382441
(43) Date of publication of application: 31.07.2019
(73) Proprietor: Cepsa S.A.U., 28046 Madrid (ES)
(72) Inventor: LARRAZ MORA, Rafael, 28046 Madrid (ES); BARRIO IRIBARREN, Izaskun, 28805 Madrid (ES); CORNEJO FERNÁNDEZ-GAO, Ana, 28805 Madrid (ES)
(74) Representative: Hoffmann Eitle
(86) International application number: PCT/EP2017/073963
(87) International publication number: WO 2018/055065

(56) References cited:
- WO-A1-2013/150457
- US-A1- 2009 270 643
- IRENE NOTAR FRANCESCO ET AL: "Simple salts of abundant metals (Fe, Bi, and Ti) supported on montmorillonite as efficient and recyclable catalysts for regioselective intramolecular and intermolecular hydroalkoxylation reactions of double bonds and tandem processes", RSC ADVANCES: AN INTERNATIONAL JOURNAL TO FURTHER THE CHEMICAL SCIENCES, vol. 6, no. 24, 1 January 2016 (2016-01-01), pages 19807-19818, XP055342203, GB ISSN: 2046-2069, DOI: 10.1039/C5RA25176A

## Description

### FIELD OF THE INVENTION

The present invention relates to the chemical field, in particular to the petrochemical field. More particularly, the invention describes the use and production of a solketal ether compound, in particular solketal-ter-amyl-ether (STAE), in diesel fuels, gasoline, and rapeseed biodiesel, with which the limited solubility of the fuel additive solketal is significantly increased and with which products such as glycerine, acetone and a light naphtha stream can be recycled to afford solketal ethers.

### BACKGROUND OF THE INVENTION

The addition of oxygen compounds to fuels in the form of alcohols and ethers has proved to be favourable. For instance, it is known that the addition of oxygen-containing compounds in fuels results in an improvement in fuel quality.

In WO 81/00721 a fuel mixture is described which has been modified by the addition of alcohols, water, ethers and vegetable oil. U.S. Patent No. 4,353,710 is also concerned with the modification of diesel fuels with ethers and esters. The addition of ethers to diesel fuels has been described in DE 3 140 382.

The improvement of diesel fuel quality by the addition of aliphatic polyethers is described in U.S. Patent No. 2,655,440. A mixture of alcohol and oxygenated hydrocarbons with a molecular weight from 250-500 was used in U.S. Patent No. 4,753,661 for improving the quality of gasoline and diesel fuel.

The invention described in U.S. Patent No. 5,308,365 relates to a low sulphur diesel fuel that contains dialkyl and trialkyl derivatives of glycerine.

The production of glycerine ethers is the focus of a number of patents and patent applications. For example, U.S. Patent No. 1,968,033 describes the etherification of multivalent alcohols. The reaction of multivalent alcohols and tertiary olefins is described in DE 4 222 183. A method for the large-scale technical production of glycerine ethers is disclosed in EP 649 829. Glycerine ethers also occur as a by-product in the separation of tertiary olefins from the C4 fraction in the distillation of crude oil. This is disclosed in U.S. Patent No. 1,968,601.

In U.S. Patent No. 4,605,787 the production of alkyl tertiary alkyl ethers is described, whereby acidic zeolites are used as catalyst systems. The glycerine ethers are also used as phase agents in the reaction of glycerine and isobutene in DE 1 224 294.

In WO 94/01389 the production of polyalkyl ethers from polyhydroxy compounds with a high molecular weight is described.

US2009/0270643 and WO2013/150457 disclose fuel compositions comprising glycerine derivatives.

The use of these glycerine ethers in fuel is intended to eliminate hydrophilicity, to reduce the boiling temperature of fuel components, and to achieve a reduction in density while maintaining the cetane number.

The disadvantage with these substances is that a glycerine-ether mixture is formed with a maximum of 11% of triethers. The remainder consists of monoethers and diethers, which because of the hydroxyl groups still present are in part not soluble in the individual fuel components.

Due to steric hindrance, 100% conversion to triethers is not possible. The conversion reaction to glycerine ethers is almost thermoneutral and heavily entropic. This leads to the situation that, as the temperature increases, the yield is reduced and oligomerisation sets in. With a reduction in the reaction temperature, however, the reaction is slowed to such an extent that no useful reaction takes place.

The production of glycerine esters is described in the GDR Patent Specification 156 803. This relates to the production of triacetin.

The esterification to lower glycerine esters does indeed bring the boiling point into the range of the diesel fuel, but with a longer acryl reside no adequate ignition behaviour is achieved. On the other hand, the boiling behaviour of the triacetin is too high and therefore cannot be used in gasoline. With glycerine esters, which lie within the boiling range of the conventional fuel components, solubility in fuels is no longer guaranteed.

The disadvantage of this substance class is due to the inadequate physical characteristics which prevent it from being used in gasoline fuels, and the deficient ignition readiness in diesel fuels.

The production of glycerine acetates is described by R. Tink et. al. (J. Techn., 29 243 (1951)) using the example of the reaction of glycerine with butyraldehyde.

Dioxalans with longer alkyl residues, the production of which is described by C. Diantadosi (J. Org. Chem., 80:6613 (1958)) are not relevant for economic reasons.

Determinant for admixture in diesel fuel, gasoline, and rapeseed biodiesel is its solubility in these fuel components. This is very problematic due to the hydroxyl group which is present, however even if the boiling point of the glycerine acetals is drastically reduced, the density in all cases is perceptibly above 1.02 g/ml. The use of these acetals in diesel fuel was disappointing due to its poor ignition behaviour.

In order to overcome these problems, methods for producing an oxygen-containing compound used as fuel additive based on a) the reaction of a multivalent alcohol with an aldehyde or ketone to produce an acetal, and b) etherification of the still free hydroxyl groups of the acetal produced in step a) with tertiary olefins have been disclosed. These methods have produced specific oxygen-containing compounds such as 2,2-dimethyl-4-hydroxymethyl-1,3-dioxalantertbutyl ether (STBE). However, as shown in the examples of the present disclosure, the solubility of many of these compounds in fuels, including STBE, failed to meet the expectations.

Therefore, there is still a need to provide an oxygen-containing compound capable of increasing fuel quality and having an improved solubility in fuels, in particular in diesel.

### BRIEF DESCRIPTION OF THE INVENTION

It was surprisingly found that the aforementioned technical problem can be solved by a composition comprising solketal-ter-amyl-ether (STAE), wherein, as shown in the examples, the solubility of STAE is significantly better than the solubility shown for STBE. In addition, it was also surprisingly found that by using the method for the production of STAE herein described, environmentally unfriendly compounds such as glycerine, acetone and in particular light naphtha can be recycled to afford solketal ethers.

In this regard, the present invention describes the use and production of a solketal ether compound, in particular solketal-ter-amyl-ether (STAE), in diesel fuels, gasoline, and rapeseed biodiesel, with which the limited solubility of the fuel additive solketal and STBE is significantly increased and whereby products such as glycerine, acetone and light naphtha can be recycled to afford solketal ethers. These improvements are achieved by a) the production of 2,2-dimethyl-4-hydroxymethyl-1,3-dioxolane (solketal) from glycerine and acetone and b) by reacting the 2,2-dimethyl-4-hydroxymethyl-1,3-dioxolane produced in step a), or obtained in any other way, with tertiary alkene compounds in order to etherify the remaining OH group.

### BRIEF DESCRIPTION OF THE FIGURES

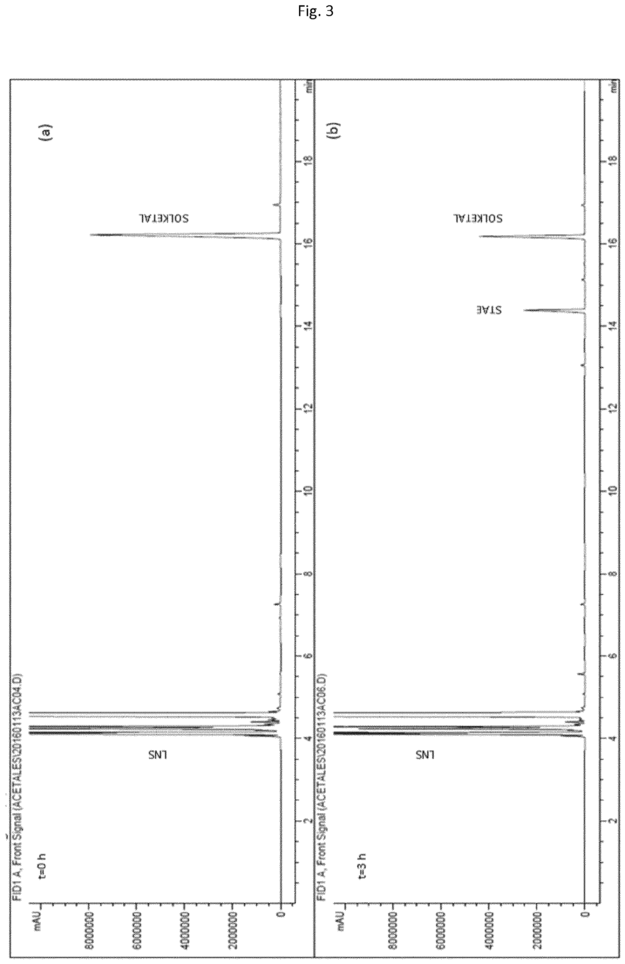

**Fig 1****. Photograph of 2% solketal in diesel (phase separation)**
   When some additives are added in concentrations above the solubilisation limit, a phase separation can be observed as demonstrated by the photograph.
**Fig. 2****. Distillation separation (quantification and purity of each distillation cut)**
   A discontinuous distillation process was carried out, and quantified using GC-FID equipment. The results are shown in this figure, where the composition of each distillation cut (% wt.) can be seen.
**Fig. 3****. GC-MS analysis of solketal etherification reaction:** (a) Initial t= 0h and (b) Final t=3h After the reaction time, new peaks can be observed in GC-MS analysis. These new compounds are STAE and its isomers.
**Fig. 4****. GC/MS-TOFF of the etherification reaction product.**
   Structural analysis of the different m/z fragment obtained from mass analysis.
   The molecular weight can be approximated to within six decimal places using this analysis. Therefore, it is possible to assert that the main signal in GC-MS chromatogram is STAE.
**Fig. 5****. Olefin: solketal space velocity and molar ratio influence on solketal conversion with amylene**
   The effect of LHSV and molar ratio in the conversion of solketal in STAE continuous production process is demonstrated.
**Fig. 6****. Olefin: solketal molar ratio feed influence on the solketal conversion with naphtha** The effect of molar ratio in conversion of solketal in STAE continuous production process with FCC naphtha is shown.
**Fig. 7****. Effect of lubricity in diesel**
   Solketal ethers are added to diesel to show their effect on lubricity.
**Fig. 8****. GC-MS analysis of solketal etherification reaction using isobutene:** (a) Initial t= 0h and (b) Final t=3h
   After the reaction time, new peaks can be observed in GC-MS analysis. These new compounds are STBE and its isomers. Compounds resulting from the polymerisation of isobutene can be observed. In contrast, Fig. 3 shows that in the formation of STAE no polymerisation products are observed and the reaction proceeds cleanly.

### DETAILED DESCRIPTION OF THE INVENTION

Glycerine, as an extremely hydrophilic substance, cannot be mixed with either gasoline or with diesel fuel and rapeseed biodiesel. The problem is thus to modify glycerine in such a way that the products can be used as fuel additive. To do this, it is considered necessary to react the hydroxyl groups present in the glycerine molecule, so that they form an acetal, followed by etherification of the hydroxyl group still present by means of a tertiary olefin. However, surprisingly such process does not necessarily result in a compound that is sufficiently miscible in fuels and as such, suitable as a fuel additive; this is the case for STBE as shown in the examples.

However, unexpectedly following a related process, the authors of the present invention found a new compound having unforeseen properties. In this regard, the authors of the invention carried out acetalization of glycerine (pharma quality) with acetone (<2000 ppm water) in a first step. This reaction takes place in the presence of an acid agent (using commercial acid resin CT 275 in this case).

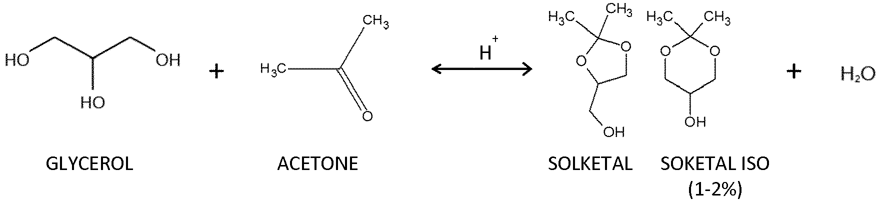

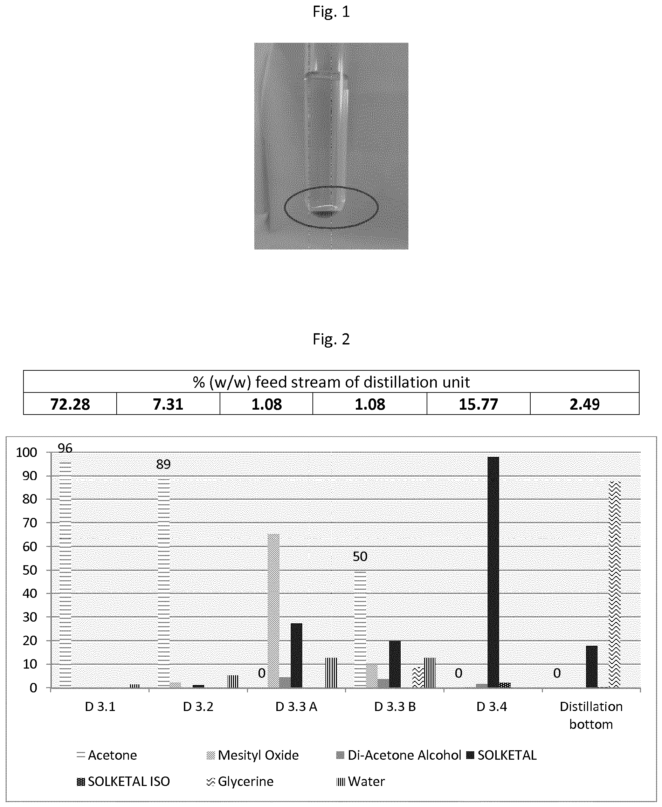

For this purpose, the authors of the present invention carried out *the following reactions:*
- *Batch reaction'.* 57.56 g of acetone and 9.13 g of glycerine were introduced in a reactor (pyrex bottles). Then, 0.457 g of resin (CT 275) were added. This was kept at a constant temperature (50°C) and under magnetic stirring (250 rpm) for 2 hours.
- *Product analysis:* The reaction effluent was analyzed by gas chromatography with a flame ionization detector (GC-FID), previously calibrated with standards for quantifying the reaction products and reagents.
- *Continuous reaction'.* The continuous reaction was carried out in a fixed bed reactor, maintaining a constant temperature of 50°C, setting flow rates (3.44 mL/min of acetone and 0.56 mL/min of glycerine), and pressure at 3.5 bar. Once the reaction started, it was considered that the steady state was achieved when the passage of 2 bed volumes of reagents through the reactor ended. After that, successive samples were taken at the reactor outlet to analyse the reaction products.
- *Fractionation of the acetalization effluent:* The reaction products were separated from the reagents in a rotavapor in 4 steps. 5 different sections were obtained (described in Figure 2). As can be seen, the section rich in solketal had a purity of greater than 95 % by weight.

Solketal etherification tests were subsequently performed with two refinery streams resulting from fluid catalytic cracking (FCC): a light naphtha stream, and another second stream, which is a light fraction of that naphtha which is separated downstream of the hydro-treating process. The experimental process was the same: batch experiments to verify reaction viability and analysis of the results to optimize conditions with regard to continuous scaling.

The batch reactions were performed in thermostated glass reactors with magnetic stirring, with a heating plate and a bath with a thermally stable fluid. The experimental method performed is described below:
- *Starting the test:* The reactor was loaded with 5.43 g of solketal, 19.89 g of amylene and 0.2715 g of catalyst. A sample was taken for analysis of the reaction mixture at zero time. The reactor was closed and it was introduced in the thermostated bath, pre-heated to 65°C. Stirring was set at 350 rpm and the reaction started.
- *Stopping the reaction:* Once the reaction time (2 hours) lapsed, the reactor was removed from the thermostated bath and said reactor was introduced in a vessel with ice to stop the reaction and to remove pressure from the reactor.
- *Product analysis:* The samples taken at the beginning and end of the reaction were analysed by gas chromatography with a flame ionization detector (GC-FID), previously calibrated for quantifying the reaction products and reagents.

For the continuous reactions, generally an installation with a fixed bed reactor, with flow rate, pressure and temperature controls that allowed maintaining the desired operating conditions was used. The method used is described below:
- *Starting the test:* With the temperature of the catalyst bed set at 65°C and the amylene and solketal feed tanks loaded, the reagent flow rates were set (5.52 mL/min of amylene and 0.88 mL/min of solketal), and the pressure was set at 3.5 bar. Once started up, the steady state was considered to have been reached after 2 *Bed Volumes* of reagents passed through the reactor. After that time, successive samples were taken at the reactor outlet to analyse the reaction products.
   *Stopping the reaction'.* Once the set sampling time (5-6 hours) lapsed, the reagent feed pumps were stopped and the reaction products were placed in cold storage.
- *Product analysis:* The samples taken over the course of the reaction were analysed by gas chromatography with a flame ionization detector (GC-FID), previously calibrated for quantifying the reaction products and reagents.

Following the working methodology established above by means of using refinery streams and in which tests were also performed with pure olefins in liquid phase at room temperature, such as **2**-methyl-**2**-butene (β-isoamylene), in reaction, the appearance of a new compound in the analysis of the reaction effluent, which could not be clearly identified by GC-MS, because the target compound is not documented in the available databases, was observed.

Given this situation, for the purpose of identifying this compound, it was assumed that, according to the reaction considered with pure olefins in liquid phase at room temperature using 2-methyl-2-butene (β-isoamylene), the target compound STAE was yielded according to the reaction shown below:

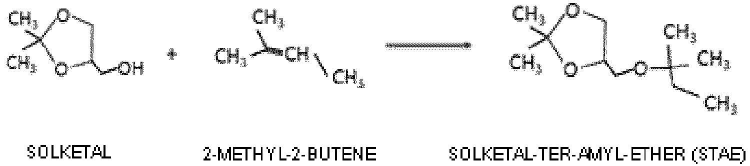

Different isomers, such as those shown below, may also be obtained, generally in a combined amount of up to 15% (w/w):

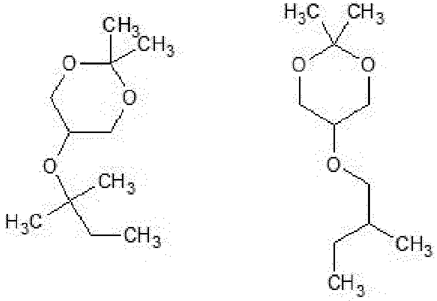

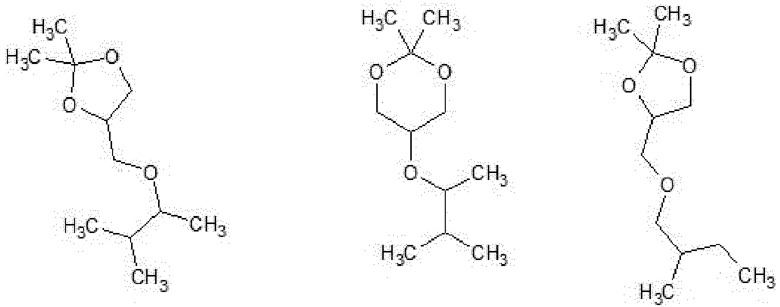

For the purpose of demonstrating this result, once the reagents were subjected to suitable operating conditions and samples of both reagents and reaction products were analysed by GC-FID, the results shown in Figure 3 were obtained.

Figure 3 shows chromatograms of the mixture of reagents (a) and of the reaction products (b). The chromatograms show both the appearance of a peak with a retention time of 14.0 to 14.5 minutes, and the disappearance of part of the solketal present in the analysis of the reagents. This result confirmed the reactivity of solketal with amylenes and the formation of a new compound **10** to be identified.

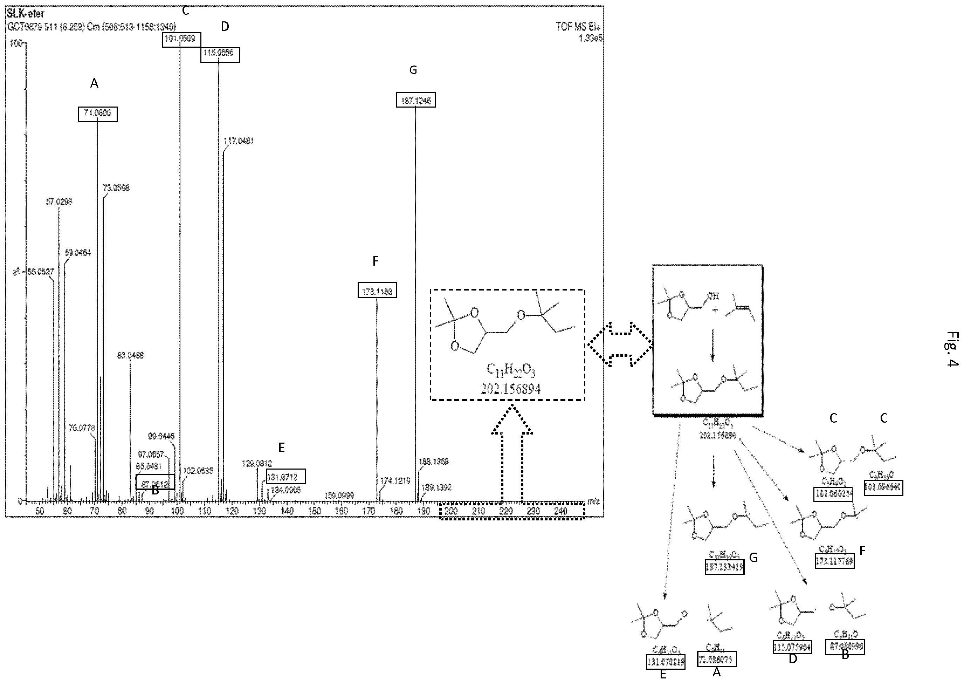

Since the target compound is not present in the available mass spectrometry databases, the GC-MS TOFF (gas chromatography coupled to a time-of-flight mass spectrometer) technique was used. The results of the analysis of the compound are shown in Figure 4.

Characteristic fragments associated with the splitting of the target molecule are highlighted in the mass spectrum that is shown, which are consistent with the fragments and the theoretical exact mass. Specifically, the exact mass is 202.1559 amu, which corresponds to molecular formula C₁₁H₂₂O₃, demonstrating that STAE is obtained.

Once the compound STAE was identified, as shown throughout the examples of the present specification, a number of assays using this specific compound were performed. Based on the information obtained thereof it was proven that a composition comprising solketal-ter-amyl-ether (STAE) is capable of increasing the quality of fuels and has an improved solubility in fuels, in particular in diesel. Moreover, the solubility of STAE in fuels was unexpectedly significantly greater than the solubility of STBE in fuels.

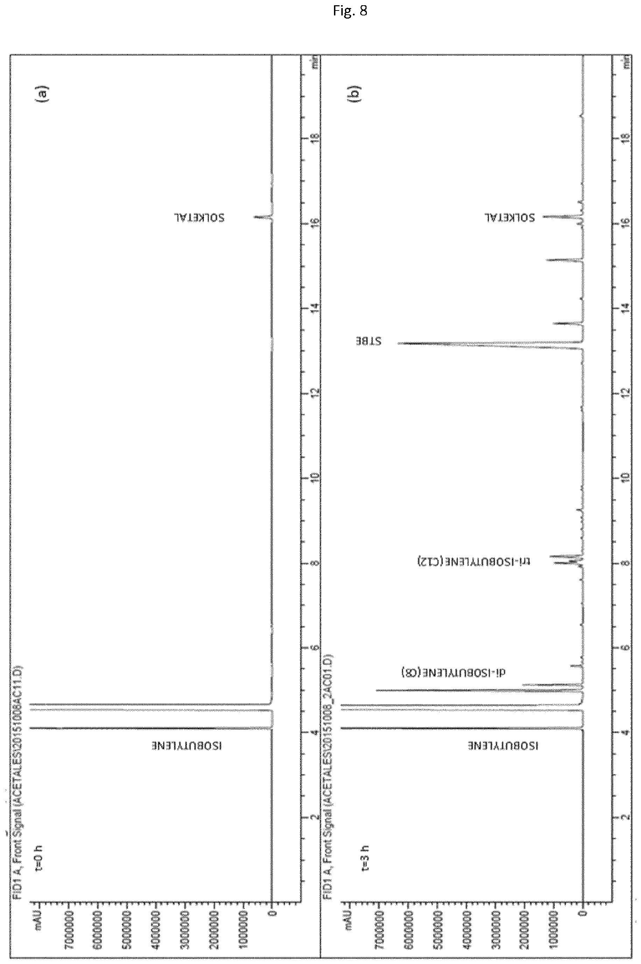

Furthermore, as shown in Figure **8****,** which depicts the chromatogram for the reaction to form STBE, dimers and trimers of isobutene were afforded. However, in the reaction to form STAE, no detectable polymerisation products of amylene were observed (see Figure 3).

In addition, it was also surprisingly found that by using the method of production of STAE, environmentally unfriendly compounds such as glycerine, acetone and in particular light naphtha can be recycled to afford solketal ethers.

Based on the above results, the present invention describes the production of a new solketal ether compound, in particular solketal-ter-amyl-ether (STAE), and its use in diesel fuels, gasoline, and rapeseed biodiesel, with which the limited solubility of the fuel additive solketal and STBE is significantly increased, and whereby products such as glycerine, acetone and light naphtha can be recycled to afford solketal ethers.

These improvements are achieved by a) the production of 2,2-dimethyl-4-hydroxymethyl-1,3-dioxolane (solketal) from glycerine and acetone and b) by reacting the 2,2-dimethyl-4-hydroxymethyl-1,3-dioxolane produced in step a), or obtained in any other way, with a specific group of amylene compounds in order to etherify the remaining OH group.

Therefore, a first aspect of the invention refers to a method for producing a solketal-ter-alkyl-ether (STRE) of formula I: 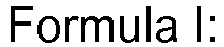 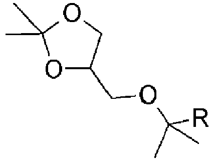 which comprises the etherification of the free hydroxyl group of acetal 2,2 dimethyl-4-hydroxymethyl-1,3-dioxolane (Solketal) with a tertiary olefin selected from the group consisting of compounds of formula II: 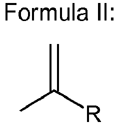 wherein R is a a C₂ to C₆ alkyl group. In a preferred embodiment, R is a C₂ to C₃ alkyl group. In one embodiment, R is a branched or unbranched C₃ alkyl group, a a branched or unbranched C₄ alkyl group, a a branched or unbranched C₅ alkyl group, a a branched or unbranched C₆ alkyl group, preferably a branched or unbranched C₃ alkyl group, In a further preferred embodiment, R is a C₂alkyl group.

In a preferred embodiment of the first aspect of the invention, the compounds of formula II are pure isolated compounds, wherein pure isolated compounds in the context of the present invention is understood as compounds having a purity of at least 60%, at least 65%, at least 70%, at least 75%, at least 80%, at least 85%, at least 90% at least 95%, preferably at least 97%, at least 98% or at least 99%.

In another preferred embodiment of the first aspect of the invention, the compounds of formula II form part of a naphtha stream composition, preferably a light naphtha stream composition, and said naphtha stream is used in the etherification step of the acetal 2,2-dimethyl-4-hydroxymethyl-1,3-dioxolane (Solketal) of the first aspect of the invention.

It is noted that in the context of the present invention, a naphtha stream composition is understood as a distillation stream of hydrocarbons comprising mainly paraffins, isoparaffins, aromatics, naphthenes and olefins in gasoline range.

In the context of the present invention, a light naphtha stream (LNS) composition is understood as a light stream of naphtha comprising mainly paraffins, isoparaffins and olefins, predominantly C₅ chains from FCC main fraction unit.

In the context of the present invention, the term "alkyl" refers to a saturated unbranched or branched monovalent hydrocarbon residue.

In another preferred embodiment of the first aspect of the invention, the method further comprises a reaction of acetone and glycerine to produce **2,2-** dimethyl-4-hydroxymethyl-1,3-dioxolane (Solketal) prior to the etherification step.

A second aspect of the invention refers to a composition comprising or consisting of a solketal-ter-alkyl-ether (S-T-R-E) of formula I: 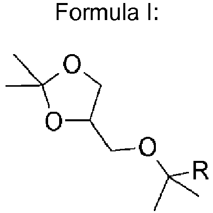 wherein R is a C**2** to C**6** alkyl group, such as a C**2** to C**5** alkyl group,
particularly a C**2** to C**4** alkyl group.

In a preferred embodiment, R is a C**2** to C**3** alkyl group. Preferably, R is a branched or unbranched C**3** group, a branched or unbranched C**4** alkyl group, a branched or unbranched C**5** group, or a branched or unbranched C**6** alkyl group, preferably a branched or unbranched C**3** group. In a further preferred embodiment, R is a C**2** alkyl group.

In a preferred embodiment of the second aspect of the invention, the formula I compound is selected from the list consisting of one or more of the following compounds:

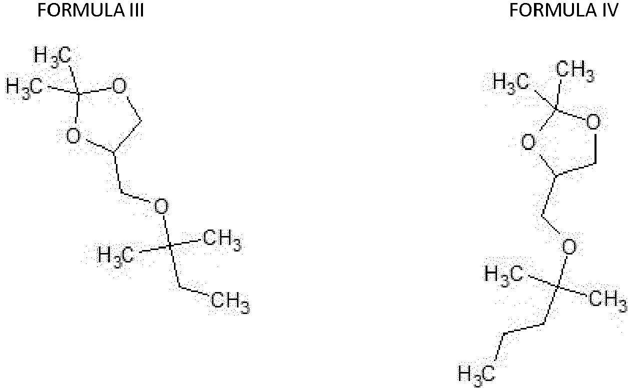

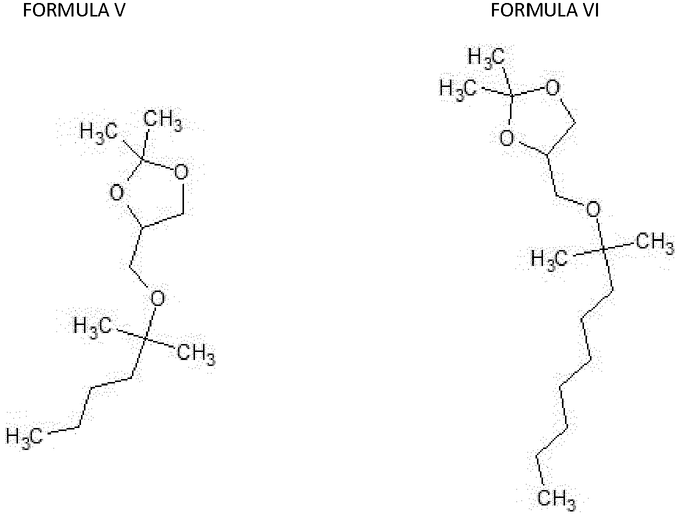

Preferably, the composition comprises or consists of the solketal-ter-amyl-ether (STAE) of formula III:

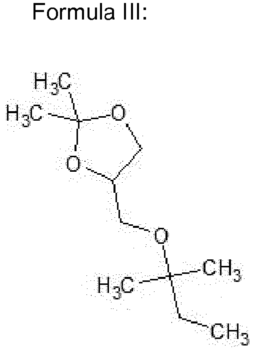

A third aspect of the invention concerns fuels, in particular diesel fuels, gasolines, or rapeseed biodiesel, comprising 0.1% by weight to maximum of 40% by weight of the completely dissolved compound according to formula I, III, IV, V or VI, or mixtures thereof.

A further embodiment of the third aspect of the invention refers to fuels, in particular diesel fuels, gasolines, or rapeseed biodiesel, comprising a compound of formula I, III, IV, V or VI, or mixtures thereof (from herein after a compound of formula I, III, IV, V or VI, or mixtures thereof shall be referred to as STAE or derivatives). Preferably said fuels comprise 0.1% by weight to 9% by weight of a compound of formula I, III, IV, V or VI, or mixtures thereof and solketal in a ratio of about 1:3 (STAE or derivatives:Solketal). Preferably said fuels comprise 0.1%, preferably 1% or 2%, by weight to 5% by weight of a compound of formula I, III, IV, V or VI, or mixtures thereof and solketal in a ratio of about 1:3 (STAE:Solketal). Preferably said fuels comprise 0.1% by weight to 90%, preferably 80%, by weight of a compound of formula I, III, IV, V or VI, or mixtures thereof to solketal in a ratio of about 1:1 (STAE or derivatives:Solketal). Preferably said fuels comprise 0.1% by weight to 50% by weight of a compound of formula I, III, IV, V or VI, or mixtures thereof to solketal in a ratio of about 1:1 (STAE or derivatives:Solketal). Preferably said fuels comprise 0.1% by weight to 90%, preferably 80%, more preferably 60%, by weight of a compound of formula I, III, IV, V or VI, or mixtures thereof to solketal in a ratio of about 3:1 (STAE or derivatives:Solketal). Preferably said fuels comprise 0.1% by weight to 50%, preferably 45%, more preferably 40%, by weight of a compound of formula I, III, IV, V or VI, or mixtures thereof to solketal in a ratio of about 3:1.

A fourth aspect of the invention refers to the use of the compounds of formula I, III, IV, V or VI, or mixtures thereof as an additive for fuels, in particular diesel fuels, gasolines, and rapeseed methyl esters. Preferably one of said compounds is combined with solketal in a ratio of about 1:3 (STAE or derivatives:Solketal), respectively, so that their combined % wt. in a fuel is between 0.1% by weight to 9% by weight. Preferably one of said compounds is combined with solketal in a ratio of about 1:3 (STAE or derivatives:Solketal), respectively, so that their combined % wt. in a fuel is between 0.1% by weight to 5% by weight. Preferably one of said compounds is combined with solketal in a ratio of about 1:1 (STAE or derivatives:Solketal), respectively, so that their combined % wt. in a fuel is between 0.1% by weight to 90%, preferably 80%, by weight. Preferably one of said compounds is combined with solketal in a ratio of about 1:1 (STAE or derivatives:Solketal), respectively, so that their combined % wt. in a fuel is between 0.1% by weight to 50% by weight. Preferably one of said compounds is combined with solketal in a ratio of about 3:1 (STAE or derivatives:Solketal), respectively, so that their combined % wt. in a fuel is between 0.1% by weight to 90%, preferably 80%, more preferably 60%, by weight. Preferably one of said compounds is combined with solketal in a ratio of about 3:1 (STAE or derivatives:Solketal), respectively, so that their combined % wt. in a fuel is between 0.1% by weight to 50%, preferably 45%, more preferably 40%, by weight.

A further embodiment of the fourth aspect of the invention concerns the use of the compound according to formula III as an additive for fuels, in particular diesel fuels, gasolines, and rapeseed methyl esters. The compound according to formula III may be preferably found in quantities from 0.1% by weight to maximum 40% by weight of the total weight of the fuel composition.

A further aspect of the invention refers to the use of a naphtha stream composition comprising a compound of formula II as defined in the first aspect of the invention, in the etherification step of the acetal 2,2-dimethyl-4-hydroxymethyl-1,3-dioxolane (Solketal) as defined in claim 1. Preferably, said naphtha stream composition is a light naphtha stream composition.

In the following, the invention will be illustrated by means of examples. However, these examples are not to be construed as limiting the invention.

### EXAMPLES

### Example 1. Reactivity of different olefins

Different olefinic compounds were used as reagents for the etherification of SLK. These compounds were selected taking into account the following aspects: chain length, double bond and methyl group position, presence or absence in naphtha and market availability.

The test conditions were: Olefin/SLK molar ratio 2-6, 50°C, 5 % wt. referred to SLK weight acid catalyst (CT-275), reaction time: 2-3 h). The results obtained are shown in Table 1. Conversion is measured by the disappearance of solketal (SLK), and selectivity is measured by the conversion to the corresponding ether.

The tests were performed in batch, using Pyrex bottles in all cases, except with isobutylene (IB), where the use of an autoclave was necessary (due to reduced isobutylene vapour pressure).

According to the results obtained, these olefins can be divided into three groups:
Group I: Linear olefins that do not significantly react (<10%) *(comparative entries 1 and 2).*
Group II: Olefins with a tertiary carbon that can react but not significantly (<10%) *(comparative entry 3 and entry 2).*
Groups III: olefins with a tertiary carbon with significant reaction *(comparative entry 4, and* 10
   *entries 1 and 3).*

**Table 1: Reaction of solketal with different olefins**

| **Entry** | **Olefin (**CAS **number)** | **Molecule** | **Product** | **Conv. (%)** | **Select. (%)** | **Presence of LLCN** |
|---|---|---|---|---|---|---|
| **Comparative entry 1** | 1-pentene (109-67-1) | 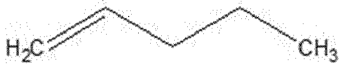 | 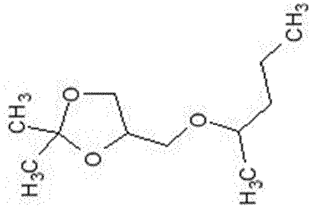 | <10 | ∼99 | No |
| **Comparative entry 2** | 1-hexene (592-41-6) | 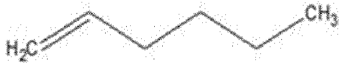 | 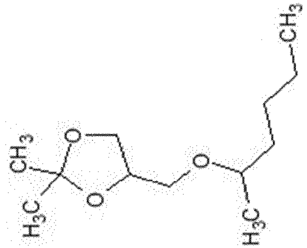 | <10 | ∼99 | No |
| **Comparative entry 3** | 2,4,4-trimethyl-2-pentene (107-40-4) | 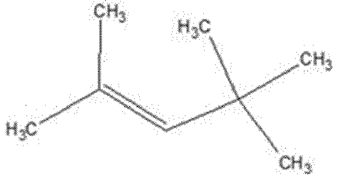 | 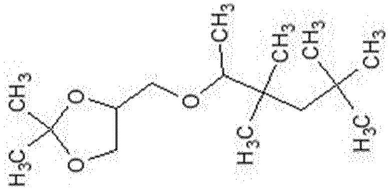 | <10 | ∼99 | No |
| **Comparative entry 4** | Methylprop ene, *Isobutylene* (115-11-7) | 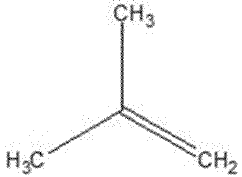 | 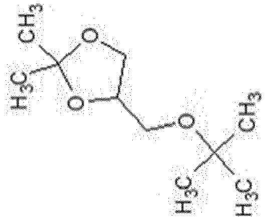 | 95 | ∼70 | No |
| **Entry 1** | 2-methyl-1-pentene (763-29-1) | 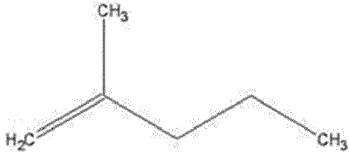 | 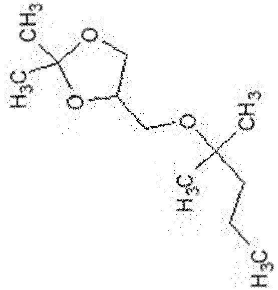 | 75 | ∼90 | No |
| **Entry 2** | 2-methyl-1,3-butadiene *(Isoprene)* (78-79-5) | 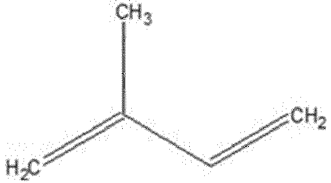 | - | - | - | No |
| **Entry 3** | 2-methyl-1-butene *(amylene)* (563-46-2) | 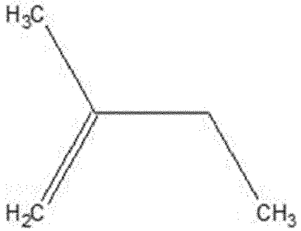 | 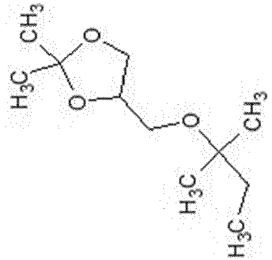 | 80 | 80 | Yes |
| **Entry 4** | 2-methyl-1-hexene (6094-02-6) | 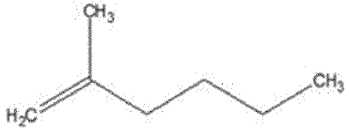 | 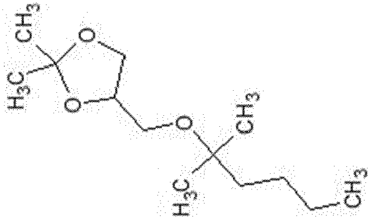 | ∼50% | ∼90 | No |
| **Entry 5** | 2-methyl-1-nonene (2980-71-4) | 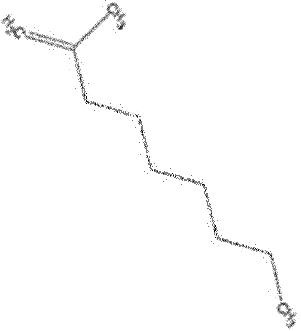 | 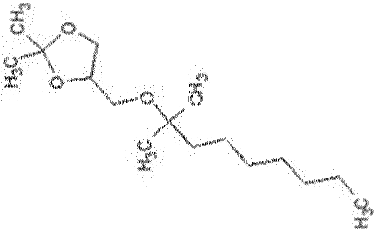 | ∼25% | ∼90 | No |

Selectivity is calculated by determining the amount of primary ether produced in relation to reacted solketal. However, it is important to note that the rest of the reacted solketal is converted into ethers (either isomers of the primary ether, or from the reaction between solketal and the dimer/trimer of the original olefin, as seen for IB). Therefore, in all cases, the selectivity with respect to the "sum of ethers" can be considered 100%.

The chromatograms of the effluents of these reactions clearly show the formation of ethers that suitably correlate with the disappearance of the reagents, except for the reaction of isoprene. In this case, the consumed isoprene is related to the formation of dimers of this olefin, whereas solketal undergoes a significant transformation, unknown until now.

The following conclusions can be drawn based on these results:
- The reactivity of olefins heavily depends on the presence of the methyl group of formula II.
- The presence of two alkyl groups bonded to the sp² hybridised carbon atom of formula II favours the reactivity of the olefin (see entry 1 and 3 and comparative entry 4).
- The presence of the two alkyl groups bonded to the sp² hybridised carbon atom of formula II is necessary but not enough for the reaction to take place in a significant manner under these conditions (see comparative entry 3 and entry 2).

### Example 2. Effect on Diesel

### 1. SOLUBILITY IN DIESEL

The solubility of a number of additives was determined by adding each of the additives to diesel and observing the final state: Completely soluble (S), which means no phase separation can be observed, and non-soluble (NS), which means phase separation can be observed (Fig. 1). Tests for solubility in diesel at 4°C were performed with the effluent of the etherification of solketal with isobutylene. Said effluent was distilled in a rotavapor, obtaining the composition shown in Table 2.

**Table 1: Composition of the additive STBE**

| **% C8** | **% C12** | **% STBE** | **%total ETHERS** | **%SLK** |
|---|---|---|---|---|
| **7.39** | 8.88 | 60.41 | 78.12 | 4% |

The solubility of SLK in diesel is as follows:

**Table 2: Results for SLK solubility in diesel at 4°C**

| | **SLK** |
|---|---|
| **(% wt.)** | |
| **0.5** | S |
| **1** | NS |

The solubility of STAE in diesel is as follows:

**Table 4: Results for STAE solubility in diesel at 4°C**

| **STAE-97*** | |
|---|---|
| **(% wt.)** | |
| **60** | S |
| **75** | S |
| **80** | S |

*The STAE has a purity of 97%. The remaining 3% consists of isomers of STAE and only 0.22% consist of non-identified compounds.

Three additives were prepared each with a different STBE/SLK ratio (1/3, 1/1 and 3/1 w/w) from 10 this effluent. In turn, these additives were added to the same diesel fuel in increasing proportions (2, 10, 20, 30 and 50 % wt.).

The results of the solubility of the additive STBE are shown in Table 5. In the case of insoluble mixtures, phase separation, as shown in Figure 1, is observed.

**Table 5: Results for STBE solubility in diesel at 4°C**

| | **STBE/SLK** | | |
|---|---|---|---|
| **(% wt.)** | **1/3** | **1/1** | **3/1** |
| **2** | S | S | s |
| **10** | NS | NS | S |
| **20** | NS | NS | S |
| **30** | NS | NS | S |
| **50** | NS | NS | S |

If these results are compared with the solubility of STAE (Table **6**) in similar conditions, it can be seen that the latter has a higher capability of solubilizing solketal in diesel.

**Table 6: Results for STAE solubility in diesel at 4°C.**

| | **STAE/SLK** | | |
|---|---|---|---|
| **(% wt.)** | **1/3** | **1/1** | **3/1** |
| **5** | S | s | s |
| **10** | NS | S | S |
| **20** | NS | S | S |
| **30** | NS | S | S |
| **50** | NS | S | S |
| **60** | | S | |
| **75** | | S | |
| **80** | | S | |
| **90** | | S | S |

### 2. SOLUBILITY AT TEMPERATURES CLOSE TO COLD FILTER PLUGGING POINT (CFPP)

Tests for solubility at temperatures close to CFPP (-5 /-7°C) were performed, obtaining the

**Table 7: STAE/SLK solubility in diesel at temperatures close to CFPP**

| **STAE/SLK** | | | |
|---|---|---|---|
| **(% wt.)** | **1/3** | **1/1** | **3/1** |
| **2** | S | S | s |
| **5** | NS | S | S |
| **10** | NS | S | S |
| **20** | NS | S | S |

**Table 8: STBE/SLK solubility in diesel at temperatures close to CFPP**

| **STBE/SLK** | |
|---|---|
| **(% wt.)** | **1/1** |
| **2** | S |
| **5** | S |
| **10** | NS |
| **20** | NS |

As can be seen, the behaviour is similar to that obtained at more moderate temperatures (around 4°C).

### 3. EFFECT ON DIESEL SPECIFICATION

The effect of the addition of these three additives (hereinafter, STBE-1/3, STBE-1/1 and STBE-3/1) on some of the diesel specification properties was measured. These can be compared with the analysis of the diesel without additives (AC67) and with the addition of 2% STAE-2.5/1 to the same diesel fuel, as shown in Table 9.

It should be noted that a base diesel with a non-compliant lubricity and distillation specification (95%) is used. A slight improvement *(*may be within the uncertainty of the method)* in the 5 lubricity of the resulting mixture can be seen. The addition of these additives also involves an increase in density, there being no significant differences in the behaviour of the different ether mixtures.

### 4. EFFECT ON LUBRICITY IN DIESEL

Lubricity was measured according to the standard method EN ISO 12156-1. The ISO standards referred to herein provide reliable, internationally recognised methods for measuring certain parameters.

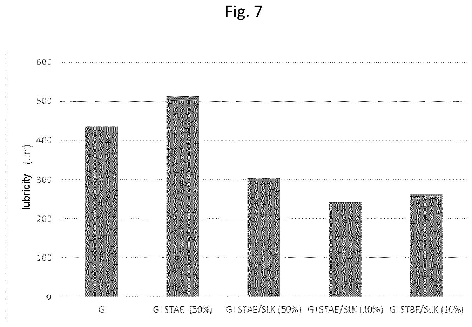

The effect on lubricity in diesel by the addition of mixtures of solketal and ethers (weight ratio 1/1) by up to 50 %wt. is shown in Figure 7.

From Figure 7, it can be seen that the addition of ethers/solketal mixtures improves lubricity in diesel, with a greater effect observed for STAE/SLK than for STBE/SLK.

**Table 9 Effect on DIESEL specification**

| **Specification** | **Unit** | **Standard limits [1]** | | **Reference** | **sTBE-1/3** | **STBE-1/1** | **STBE-3/1** | **STAE.2.5/1** |
|---|---|---|---|---|---|---|---|---|
| | | **min** | **max** | **AC67 (target)** | **AC67-2%** | **AC67-2%** | **AC67-2%** | **AC67-2%** |
| **Cloud point** (EN 23015) | °C | - | - | -3 | -2 | -3 | -3 | -3 |
| **Density at 15°C** (EN ISO 12185) | Kg/m3 | 820 | 845 | 842.6 | 844.6 | 843.7 | 844.2 | 844.2 |
| **DISTILLATION** (EN ISO 3405) | | | | | | | | |
| 65% Vol. | °C | 250 | | 304.4 | 305 | 305.9 | 304.6 | 304.9 |
| 85% Vol. | °C | - | 350 | 341.5 | 340.3 | 341.5 | 337.7 | 339.8 |
| 95% Vol. | °C | - | 360 | 363.8 | 363.1 | 364.5 | 359.7 | 364.1 |
| 10% Vol. | °C | - | - | 195 | 197 | 193 | 201 | 198 |
| 50% Vol. | °C | - | - | 281 | 283 | 281 | 282 | 282 |
| 90% Vol. | °C | - | - | 352 | 351 | 352 | 348 | 351 |
| **Cetane number (EN ISO 4264)** | | 46 | • | 50.6 | 50.2 | 50 | 50.8 | 50.3 |
| **Kinematic visc. at 40°C** (EN ISO 3104) | mm³/s | 2 | 4.5 | 3.067 | 3.052 | 3.022 | 3.022 | 3.025 |
| **Flash point** (EN ISO 2719) | °C | 55 | | 61.5 | 57.5 | 57.5 | 60 | 59 |
| **CFPP** (EN 116) | °C | - | - | -7 | -4 | -5 | -4 | -4 |
| **Lubricity** (EN ISO 12156-1) | µm | | 460 | 537 | 480 | 437 | 438 | 473 |

| | | | | | | | | |
|---|---|---|---|---|---|---|---|---|
| [1] Boletín Oficial del Estado. Nûm.215. Sec I. Pág 76436-76445 | | | | | | | | |

From the data shown in Example 2, the following conclusions can be drawn:
- The addition of SLK is limited to 0.5 % by weight. The addition of STAE is not limited by its solubility with diesel.
- The use of STAE and STBE improve the solubility of SLK, but to a higher degree with STAE.
- The use of additives comprising SLK and ethers could improve the lubricity of diesel.

### Example 3. Effect on Gasoline

### 1. SOLUBILITY/STABILITY IN GASOLINE

Solubility and stability tests were performed by mixing different additives in gasoline in several proportions and observing their behaviours (at 4°C). Where no phase separation appears, the additives can be considered to be completely soluble and stable (S). Where the additive is insoluble (NS) two phases can be observed. Furthermore, in some cases, instability (IS) can be observed; indicated by the presence of glycerol drops appearing at the bottom of the mixtures. Tests for solubility and stability of the STAE/SLK and STBE/SLK mixtures at a temperature of 4°C in gasoline were performed. The results are shown in the following table.

**Table 10: STAE/SLK and STBE/SLK stability/solubility test in gasoline 95 at 4°C**

| | **STAE/SLK** | | | **STBE/SLK** | | |
|---|---|---|---|---|---|---|
| **(% wt.)** | **1/3** | **1/1** | **3/1** | **1/3** | **1/1** | **3/1** |
| **2%** | S | S | S | IS | IS | IS |
| **10%** | S | S | S | IS | IS | IS |
| **20%** | S | S | S | | | |
| **30%** | S | S | S | | | |

As can be seen from these results, there are no issues with solubility and stability in any of the tested proportions in the case of STAE and SLK. In those gasolines containing STBE as an additive, the ether is completely soluble. However, instability is observed when STBE was employed as indicated by the presence of some glycerine drops at the bottom of the mixtures. From this it can be inferred that there is a reversal of the acetalization-etherification reactions. The presence of these drops make the mixtures NON-COMPLIANT as regards appearance, as required in the standard and seen in the following section

### 2. EFFECT ON GASOLINE SPECIFICATION

Gasolines containing STAE/SLK and STBE/SLK mixtures, as additives, in a 1/1 ratio, in two different proportions (**2** and **10**%), were characterized, obtaining the results shown below.

**Table 11: Effect on gasoline specification**

| | | | | | | | | | |
|---|---|---|---|---|---|---|---|---|---|
| **Specification** | **Unit** | **Commercial Specification** | | | **Reference Gasoline (AC116)** | **Reference Gasoline +2% STBE 1/1** | **Reference Gasoline +10% STBE 1/1** | **Reference Gasoline +2% STAE 1/1** | **Reference Gasoline +10%STAE 1/1** |
| | | Season | min | max | 55.9 | 55.5 | **53.3** | 56.2 | **53.8** |
| **Vapour Pressure (EN13016-1)** | kPa | summer | 45 | 60 | | | | | |
| | | winter | 50 | 74 | | | | | |
| **RON (ASTM 0 2700)** | | | 95 | 97.9 | 95.2 | 95.8 | 96.0 | 95.0 | 95.0 |
| **DISTILLATION (EN ISO 3405)** | | | | | | | | | |
| **70°C Cut** | %v/v | summer | 20 | 45 | 31.0 | 31.0 | 31.0 | 31.9 | 29.6 |
| | | winter | 22 | 47 | | | | | |
| **100°C Cut** | %v/v | summer | 46 | 69 | 47.7 | 47.5 | 47.7 | 47.8 | 45.0 |
| | | winter | | | | | | | |
| **150°C Cut** | %v/v | - | 75 | - | 85.8 | 85.1 | 85.8 | 85.1 | 80 |
| **FBP** | °C | - | - | 210 | 205.1 | 201.8 | 205.2 | 27.8 | 209.1 |
| **Residue** | %v/v | - | - | 2 | 1 | 0.8 | 1 | 0.9 | 0.9 |
| **OXIDATION STABILITY (EN ISO 7536)** | min | - | - | - | <500 | <500 | <500 | <500 | <500 |
| **GUMS (EN ISO 6246)** | Mg/100ml | - | - | 5 | <1 | <1 | <1 | <1 | <1 |
| **Cu CORROSION (EN ISO 2160)** | **EscASTM** | - | - | 1b | 1a | 1a | 1a | 1a | 1a |
| **APPEARANCE (VISUAL INSPECTION** | °C | | | | COMPLIANT | NON-COMPLIANT | NON-COMPLIANT | COMPLIANT | COMPLIANT |

*As can be seen in Table 11, the addition of these compounds does not significantly affect the gasoline specification at the tested proportions, with the exception of vapour pressure, where it can be seen that adding 10% of both ethers results in a drop in said parameter.*

In addition, in the case of STBE, the result NON-COMPLIANT is obtained for the appearance due to drops formed at the bottom of the mixture.

In conclusion, it is clear that the effect of the addition of these compounds does not significantly affect the main properties of gasoline, except for a slight improvement in vapour pressure. However, instability occurs when STBE/SLK is added to gasoline.

### Example 4. Obtaining Solketal-ter-amyl-ether in a continuous process from pure reagents

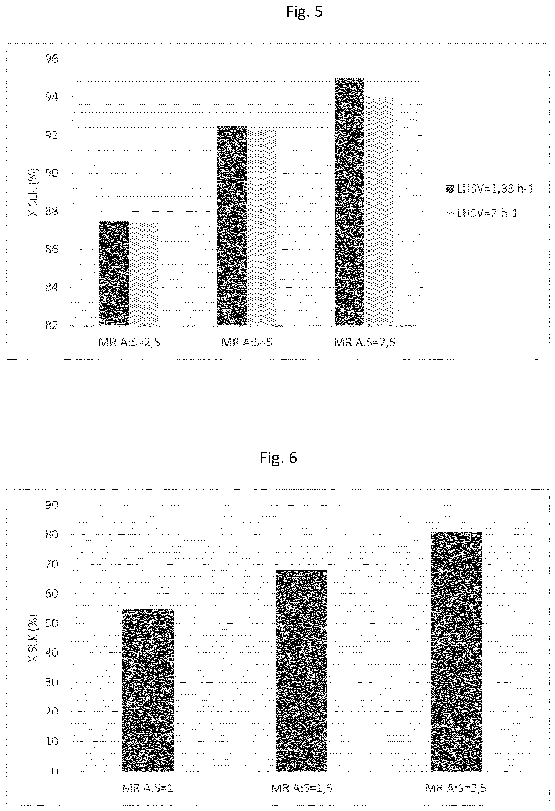

Figure 5 shows the results obtained in the etherification reaction of solketal (98%, Sigma Aldrich ref. 122696) with 2-methyl-2-butene (95%, Sigma Aldrich ref: 86262).

Figure 5 shows the influence of both the amylene:solketal molar ratio and the spatial velocity (LHSV) in the conversion of solketal. Firstly, it can be seen that the studied spatial velocity has no effect on the conversion of the reaction, which is consistent with the optimal operating band of resins of this type for reactions of this type (LHSV between 1 and 5 h⁻¹). However, a greater conversion to higher amylene:solketal molar ratios is observed, reaching a maximum conversion of solketal of 95%, with a molar ratio of 7.5:1 and an LHSV of 2 h⁻¹.

### Example 5. Obtaining Solketal-ter-amyl-ether in a continuous process with FCC naphthas

Once the target compound (STAE) is synthesized and identified, experiments with refinery streams rich in C₅ hydrocarbons, including amylenes 2-methyl-1-butene and 2-methyl-2-butene, were conducted. Among the streams tested, a desulfurised light cracked naphtha (LNS) with an average total amylene content of 20% was selected. Figure 6 shows average conversions obtained with different amylene:solketal molar ratios.

The results obtained are consistent with the results obtained with 2-methyl-2-butene of Sigma Aldrich. An increase in the average conversion of the reaction by increasing the amylene:solketal molar ratio is observed, with a maximum average conversion of 83% in a reaction with an amylene:solketal molar ratio of 2.5:1 and an LHSV of 2 h"¹.

The results obtained show that the final product obtained in the process, STAE, is independent of whether the amylene is in the form of 2-methyl-1-butene or 2-methyl-2-butene. Without being bound by a particular theory, it appears that these two amylenes are present in some sort of equilibrium as discussed in Lixin et al., Petroleum Science and Technology, 2008, 575-585*,,* and that this may play a role in the consistency of obtaining STAE as the final product.

## Claims

1. Method for producing a solketal-ter-alkyl-ether (STRE) of formula I: 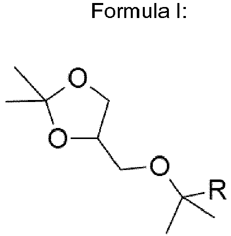 which comprises the etherification of the still free hydroxyl groups of the acetal 2,2-dimethyl-4-hydroxymethyl-1,3-dioxolane(Solketal) with tertiary olefins selected from the group consisting of compounds of formula II: 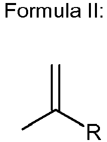 wherein R is a C₂ to C₆ alkyl group.

2. The method of claim 1, wherein R is a C₂ to C₃ alkyl group.

3. The method of claim 1, wherein R is an ethyl group.

4. The method of any of the preceding claims, wherein the compounds of formula II are pure isolated compounds.

5. The method of any of claims 1 to 3, wherein the compounds of formula II form part of a naphtha stream composition, preferably a light naphtha stream composition, and wherein said naphtha stream is used in the etherification step of the acetal 2,2-dimethyl-4-hydroxymethyl-1,3-dioxolane (Solketal) of claim 1.

6. The method of any of the preceding claims which further comprises a reaction of acetone and glycerine to produce 2,2-dimethyl-4-hydroxymethyl-1,3-dioxolane (Solketal) prior to the etherification step.

7. A composition comprising a compound of formula I: 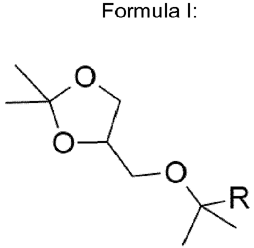 wherein R is a C₂ to C₆ alkyl group.

8. The composition of claim 7 comprising the solketal-ter-amyl-ether (STAE) of formula III:

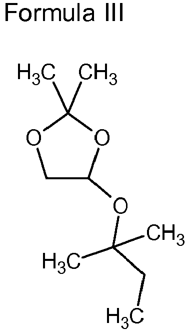

9. The composition of claim 7 comprising one or more of the following compounds:

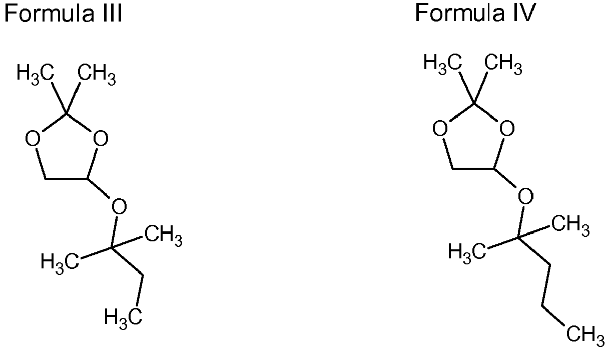

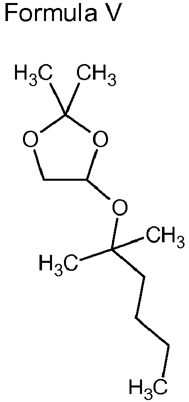

10. Fuels, in particular diesel fuels, gasolines, or rapeseed biodiesel, comprising the compound of any one of claims 7-9.

11. Fuels, in particular diesel fuels, gasolines, or rapeseed biodiesel, comprising 0.1 % by weight to maximum 90% by weight of the completely dissolved compound of any one of claims 7-9.

12. Use of the compound according to any one of claims 7-9 as an additive for fuels, in particular diesel fuels, gasolines, and rapeseed methyl esters.

13. The use of claim 12, wherein the compound is found in quantities from 0.1% by weight to maximum 90% by weight.

14. Use of a naphtha stream composition comprising the compound of formula II as defined in any of claims 1 to 3 in the etherification step of the acetal 2,2-dimethyl-4-hydroxymethyl-1,3- dioxolane (Solketal) as defined in claim 1.

15. The use of claim 14, wherein the naphtha stream composition is a light naphtha stream composition.

## Patentansprüche

1. Verfahren zur Herstellung eines Solketal-tert.-alkyl-ethers (STRE) der Formel I: 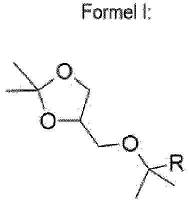 welches die Veretherung der noch freien Hydroxylgruppen des Acetals 2,2-Dimethyl-4-hydroxymethyl-1,3-dioxolan (Solketal) mit tertiären Olefinen ausgewählt aus der Gruppe bestehend aus Verbindungen der Formel II: 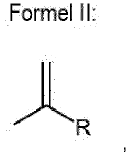 umfasst,
wobei R eine C₂- bis C₆-Alkylgruppe ist.

2. Verfahren nach Anspruch **1**, wobei R eine C₂- bis C₃-Alkylgruppe ist.

3. Verfahren nach Anspruch 1, wobei R eine Ethylgruppe ist.

4. Verfahren nach einem der vorhergehenden Ansprüche, wobei die Verbindungen der Formel II pure isolierte Verbindungen sind.

5. Verfahren nach einem der Ansprüche 1 bis 3, wobei die Verbindungen der Formel II Bestandteil einer Naphtha-Stromzusammensetzung, vorzugsweise eine leichte Naphtha-Stromzusammensetzung sind, und wobei der genannte Naphtha-Strom im Veretherungsschritt des Acetals 2,2-Dimethyl-4-hydroxymethyl-1,3-dioxolan (Solketal) nach Anspruch 1 verwendet wird.

6. Verfahren nach einem der vorhergehenden Ansprüche, welches zusätzlich eine Reaktion von Aceton und Glycerin, um 2,2-Dimethyl-4-hydroxymethyl-1,3-dioxolan (Solketal) herzustellen, vor dem Veretherungsschritt umfasst.

7. Zusammensetzung umfassend eine Verbindung der Formel I: 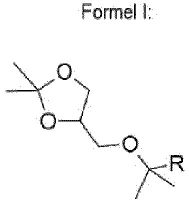 wobei R eine C₂- bis C₆-Alkylgruppe ist.

8. Zusammensetzung nach Anspruch 7, umfassend den Solketal-tert.-amyl-ether (STAE) der Formel III:

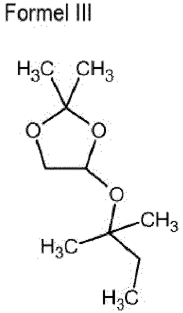

9. Zusammensetzung nach Anspruch 7, umfassend einer oder mehrere der folgenden Verbindungen:

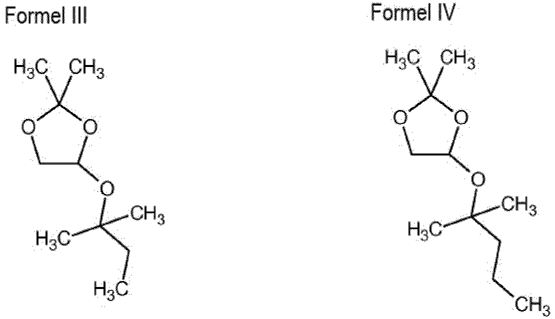

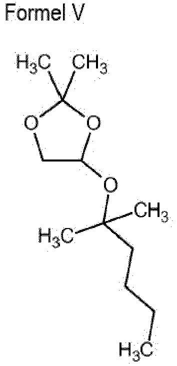

10. Kraftstoffe, insbesondere Dieselkraftstoffe, Benzine oder Rapsbiodiesel, umfassend die Verbindung nach einem der Ansprüche 7-9.

11. Kraftstoffe, insbesondere Dieselkraftstoffe, Benzine oder Rapsbiodiesel, umfassend 0,1 Gew.-% bis maximal 90 Gew.-% der vollständig gelösten Verbindung nach einem der Ansprüche 7-9.

12. Verwendung der Verbindung nach einem der Ansprüche 7-9 als Zusatzstoff für Kraftstoffe, insbesondere Dieselkraftstoffe, Benzine und Rapsmethylester.

13. Verwendung nach Anspruch 12, wobei die Verbindung in Mengen von 0,1 Gew.-% bis maximal 90 Gew.-% gefunden wird.

14. Verwendung einer Naphtha-Stromzusammensetzung umfassend die Verbindung der Formel II, wie in einem der Ansprüche 1 bis 3 definiert wird, im Veretherungsschritt des Acetals 2,2-Dimethyl-4-hydroxymethyl-1,3-dioxolan (Solketal), wie in Anspruch 1 definiert wird.

15. Verwendung nach Anspruch 14, wobei die Naphtha-Stromzusammensetzung eine leichte Naphtha-Stromzusammensetzung ist.

## Revendications

1. Procédé pour produire un tert-alkyl éther de solketal (STRE) de formule I : 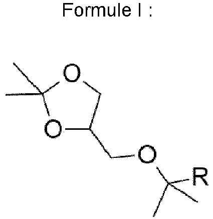 qui comprend l'éthérification des groupes hydroxyle encore libres de l'acétal 2,2-diméthyl-4-hydroxyméthyl-1,3-dioxolane (solketal) avec des oléfines tertiaires sélectionnées du groupe qui consiste en composés de formule II : 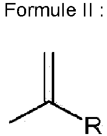 dans laquelle R est un groupe alkyle C₂ à C₆.

2. Procédé selon la revendication 1, dans lequel R est un groupe alkyle C₂ à C₃.

3. Procédé selon la revendication 1, dans lequel R est un groupe éthyle.

4. Procédé selon l'une quelconque des revendications précédentes, dans lequel les composés de formule II sont des composés isolés purs.

5. Procédé selon l'une quelconque des revendications 1 à 3, dans lequel les composés de formule II font partie d'une composition de courant de naphta, préférablement une composition de courant de naphta léger, et dans lequel ledit courant de naphta est utilisé dans l'étape d'éthérification de l'acétal de 2,2-diméthyl-4-hydroxyméthyl-1,3-dioxolane (solketal) selon la revendication 1.

6. Procédé selon l'une quelconque des revendications précédentes, qui comprend en outre une réaction entre acétone et glycérine pour produire 2,2-diméthyl-4-hydroxyméthyl-1,3-dioxolane (solketal) avant l'étape d'éthérification.

7. Composition qui comprend un composé de formule I : 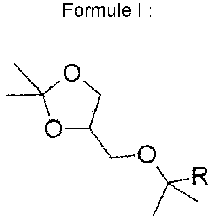 dans laquelle R est un groupe alkyle C₂ à C₆.

8. Composition selon la revendication 7, qui comprend le tert-amyl éther de solketal (STAE) de formule III :

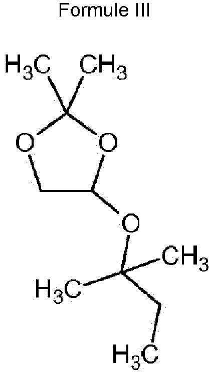

9. Composition selon la revendication 7, qui comprend un ou plusieurs des composés suivants :

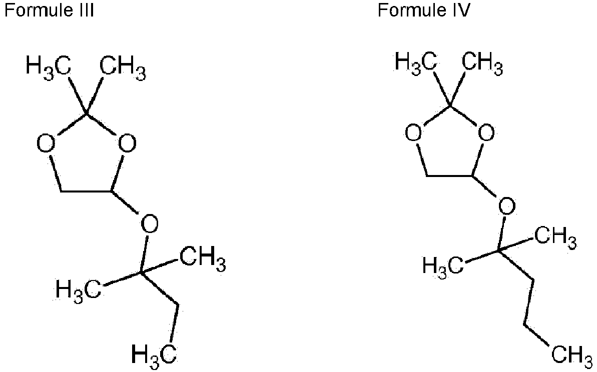

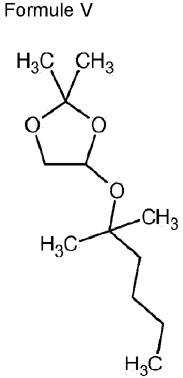

10. Combustibles, en particulier combustibles diesel, essences ou biodiesel de colza, qui comprennent le composé selon l'une quelconque des revendications 7-9.

11. Combustibles, en particulier combustibles diesel, essences ou biodiesel de colza, qui comprennent de 0,1% en poids à un maximum de 90% en poids du composé complètement dissous selon l'une quelconque des revendications 7-9.

12. Utilisation du composé selon l'une quelconque des revendications 7-9, comme additif pour combustibles, en particulier combustibles diesel, essences et esters méthyliques de colza.

13. Utilisation selon la revendication 12, dans laquelle le composé se trouve en quantités allant de 0,1% en poids jusqu'à un maximum de 90% en poids.

14. Utilisation d'une composition de courant de naphta qui comprend le composé de formule II selon l'une quelconque des revendications 1 à 3, dans l'étape d'éthérification de l'acétal 2,2-diméthyl-4-hydroxyméthyl-1,3-dioxolane (solketal) selon la revendication 1.

15. Utilisation selon la revendication 14, dans laquelle la composition de courant de naphta est une composition de courant de naphta léger.
